# EUROPÄISCHE PATENTSCHRIFT

(11) **EP 3 716 806 B1**
(45) Veröffentlichungstag und Bekanntmachung des Hinweises auf die Patenterteilung: **23.02.2022**
(21) Anmeldenummer: 18833395.9
(22) Anmeldetag: 27.11.2018
(51) Int. Cl.: A43B 3/12, A43B 7/14, A43B 7/26, A43C 11/16, A61F 5/01

(54) **HALLUX-VALGUS-SANDALE MIT ZUMINDEST EINEM GROSSZEHENSCHLAUFENABSCHNITT UND EINEM HALTESCHLAUFENABSCHNITT**
HALLUX VALGUS SANDAL WITH AT LEAST ONE HALLUX LOOP SECTION AND A HOLDING LOOP SECTION
SANDALE POUR HALLUX VALGUS COMPRENANT AU MOINS UNE SECTION À BOUCLE POUR LE GROS ORTEIL ET UNE SECTION À BOUCLE DE MAINTIEN

(30) Priorität: 27.11.2017 DE 202017107165 U; 24.01.2018 DE 102018201113
(43) Veröffentlichungstag der Anmeldung: 07.10.2020
(73) Patentinhaber: Hallufix AG, 82031 Grünwald (DE)
(72) Erfinder: BRASS, Manfred, 82031 Grünwald (DE); FISCHER, Franz, 92224 Amberg (DE)
(74) Vertreter: Molnia, David
(86) Internationale Anmeldenummer: PCT/EP2018/082679
(87) Internationale Veröffentlichungsnummer: WO 2019/102033

(56) Entgegenhaltungen:
- DE-B- 1 261 778
- DE-C- 855 612
- US-A1- 2011 130 695
- US-A1- 2011 179 674
- US-A1- 2014 123 440

## Beschreibung

Die Erfindung betrifft eine Hallux-Valgus-Sandale gemäß dem Oberbegriff von Anspruch 1, bei welcher ein Großzehenschlaufenabschnitt und ein Halteschlaufenabschnitt vorgesehen sind, und vorzugsweise ein Sohlenelement mit einer Laufsohle und einem Fußbett, ein Großzehenschlaufenabschnitt zur lateralen Lagefestlegung des Großzehs und ein Mittelfußschlaufenabschnitt vorgesehen sind, welcher beim Tragen der Sandale die Fußmittelknochen eines auf dem Sohlenelement getragenen Fußes zirkulär umschließt, wobei die Umschlingungslänge veränderbar ist.

Derartige Hallux-Valgus-Sandalen sind bekannt. In US 2011/179674 A1 wird ein Hallux-Valgus-Sandale gemäß dem Oberbegriff von Anspruch 1 beschrieben. In US 2016/0242508 A1 ist eine therapeutische Hallux-Valgus-Sandale zum Einsatz von Fehlstellungen zumindest von Großzehen beschrieben, welche ein Sohlenelement mit einer Laufsohle und einem beim Tragen an einem Fuß dem Fuß zugeordneten Fußbett und mit einem am Sohlenelement fixierten Halteschlaufenabschnitt zum Halten der Sandale am Fuß sowie einem Großzehenschlaufenabschnitt aufweist. Der Großzehenschlaufenabschnitt ist mit einem ersten Endbereich in einem zwischen dem Großzeh und dem benachbarten Zeh gelegenen Bereich des Sohlenelements am oder im Sohlenelement verankert und in seiner Umschlingungslänge um den Großzeh derart veränderbar, dass eine einstellbare Korrekturkraft auf den Großzeh ausübbar ist. Mit der Verstellung des Großzehenschlaufenabschnitts wird auch der im Mittelfußbereich angeordnete Halteschlaufenabschnitt verstellt, da beide Verstellschlaufen miteinander gekoppelt sind. Ähnliche Konstruktionen von therapeutischen Sandalen sind auch in US 3 066 678 und US 3 275 002 beschrieben. Von diesem Stand der Technik geht die Erfindung aus.

Diese Sandalen gemäß dem Stand der Technik haben den Nachteil, dass der Großzehenschlaufenabschnitt unmittelbar am Großzeh quer über diesen geführt wird. Dies erlaubt nur eine geringe Verstellwirkung, da der Angriffspunkt der Kraft ungünstig liegt, was ein geringes Drehmoment auf den Zeh bezüglich des Großzehengrundgelenks nachsichzieht. Beim Straffen der Großzehenschlaufe erfolgt auch zwangsläufig eine Rotation des Großzehs um seine Längsachse, was besonders nachteilig ist. Zudem begrenzt die Größe des Großzehenschlaufenabschnitts im Bereich der Großzehe, insbesondere an deren Eintrittsposition in die Sohlenbereich der Sandale, den möglichen seitlichen Verschiebebereich für den Großzeh. Beim Hallux Valgus geht das Krankheitsbild sowohl mit einer Achsverschiebung als auch mit einer Rotation des Großzehs einher. Die Sandalen gemäß dem Stand der Technik verstärken beide negativen Aspekte gerade noch.

In WO 2013/160377 A1 ist eine Hallux-Valgus-Sandale mit einem Sohlenelement mit einer Laufsohle und einem Fußbett beschrieben. Die Sandale weist des Weiteren ein Großzehen-Widerlager zur lateralen Abstützung des Großzehs und einen Mittelfußgurt auf, welcher beim Tragen der Sandale im Zusammenspiel mit dem Sohlenelement die Fußmittelknochen zirkulär umschließt und welcher in seiner Länge veränderbar ist. An der Innenseite der Sandale ist im Bereich des Mittelfußes ein Druckkissen vorgesehen, welches durch Anlegen des Mittelfußgurtes über das Druckkissen eine lateral gerichtete Kraft auf den inneren Mittelfußknochen ausübt. Der Mittelfußgurt ist geteilt, so dass durch den Grad der gegenseitigen Überlappung der beiden Teile des Mittelfußgurtes die zirkuläre Länge des Mittelfußgurtes einstellbar ist. Durch die durch das Druckkissen auf den auf der Fußinnenseite liegenden Mittelfußknochen ausgeübte Kraft, wodurch der entsprechende Mittelfußknochen nach außen gedrückt wird, erfährt der Großzeh eine stärkere Ausrichtung nach innen. Der Großzehengurt allerdings ist unverstellbar und fest und dient lediglich der lateralen Abstützung des Großzehs. Die Korrektur der Ausrichtung des Großzehs erfolgt damit indirekt durch die Korrektur des Mittelfußknochens, welcher durch das Druckkissen bewirkt wird. Eine zusätzliche, auch einer therapeutischen Wirkung dienende Einrichtung zur Korrektur der Fehlstellung des Großzehs erfolgt dabei direkt nicht.

Die der Erfindung zugrundeliegende Aufgabe besteht darin, eine Hallux-Valgus-Sandale bereitzustellen, bei welcher sowohl ein Großzehenschlaufenabschnitt als auch ein Mittelfußschlaufenabschnitt jeweils eine therapeutische Beeinflussung der Ausrichtung sowohl der Großzehe als auch von Mittelfußknochen ermöglichen.

Diese Aufgabe wird durch eine Hallux-Valgus-Sandale mit den Merkmalen gemäß dem Anspruch 1 gelöst. Zweckmäßige Weiterbildungen sind in den jeweiligen abhängigen Ansprüchen definiert.

Bei einer therapeutischen Behandlung einer derartigen Fehlstellung eines Großzehs wird im Verlaufe der Behandlung, wenn denn diese positiv verläuft, allmählich der Großzeh wieder in seine ursprüngliche, d. h. in Richtung des Fußes ausgerichtete Richtung, korrigiert. Während der Behandlung verändert sich also die erforderliche Korrekturkraft, um den Großzeh aus seiner nach außen gerichteten Fehlstellung in eine Geradstellung zu korrigieren. Damit dies entsprechend dem Therapieerfolg korrigierbar ausgeführt werden kann, weist der Großzehenschlaufenabschnitt einen ersten Endbereich auf, welcher am oder im Sohlenelement verankert ist. Vorzugsweise ist der Großzehenschlaufenabschnitt nun so ausgebildet, dass an seinem der Verankerung gegenüberliegenden Ende eine Kraft angreift, welche dafür sorgt, dass die Umschlingungslänge des Großzehenschlaufenabschnittes um den Großzeh derart veränderbar ist, dass eine einstellbare Korrekturkraft auf den Großzeh ausübbar ist. Dazu wird das gegenüber der Verankerung des Großzehschlaufenabschnittes liegende zweite Ende in seiner Position so verändert, vorzugsweise gezogen, dass die Umschlingungslänge verkürzt wird, so dass die entsprechende Korrekturkraft auf den Großzeh ausgeübt werden kann.

Gemäß Anspruch 1 ist der Großzehenschlaufenabschnitt vom ersten Endbereich ausgehend über den Bereich des Großzehs schräg nach hinten geführt und tritt auf der Innenseite der Hallux-Valgus-Sandale in einen zur Außenseite der Hallux-Valgus-Sandale führenden Kanal im Sohlenelement ein, wobei der Kanal im Mittelfußbereich der Hallux-Valgus-Sandale angeordnet ist.

Diese Führung des Großzehenschlaufenabschnitts verhindert ein Verdrehen des Großzehs um seine Längsachse bei Straffung der Großzehenschlaufe. Zudem bewirkt der schräge Verlauf eine Verlagerung des Kraftangriffspunktes am Großzeh und damit eine Verlängerung des Hebelarms im Vergleich zu Ausführungsformen gemäß dem Stand der Technik. Durch die Führung des Großzehenschlaufenabschnitts hinter den Großzehenballen in einen im Mittelfußbereich liegenden Kanal im Sohlenelement wird das Sohlenelement nur minimal geschwächt. Insbesondere tritt keine Schwächung im Bereich des Großzehenballens auf, bei welchem der Druck auf das Sohlenelement besonders groß ist. Durch diese erfindungsgemäße Ausbildung werden - anders als bei Sandalen gemäß dem Stand der Technik - also einer Achsverschiebung und einer Rotation des Großzehs um seine Längsachse entgegengewirkt.

Zusätzlich zu dem in seiner Umschlingungslänge einstellbaren Großzehenschlaufenabschnitt ein Mittelfußschlaufenabschnitt vorgesehen, welcher an seinen jeweiligen Endbereichen am oder im Sohlenelement befestigt ist und optional dessen Umschlingungslänge um den Mittelfußbereich derart veränderbar ist, dass eine einstellbare Korrekturkraft auf den Mittelfußbereich ausübbar ist. Dadurch, dass vorzugsweise eine Einstellbarkeit für die Umschlingungslänge des Mittelfußschlaufenabschnittes gegeben ist, kann auf den oder die Mittelfußknochen eine entsprechende Korrekturkraft ausgeübt werden, so dass die die Mittelfußknochen in ihrer Fehlstellung korrigierende Korrekturkraft gemäß den anatomischen Gegebenheiten zusätzlich zum in seiner Umschlingungslänge einstellbaren Großzehenschlaufenabschnitt eine therapeutische Wirkung des Hallux-Valgus erzielen kann.

Der Vorteil einer derartigen Vorrichtung besteht darin, dass die Umschlingungslänge des Großzehenschlaufenabschnittes und die des Mittelfußschlaufenabschnittes einzeln für die Ausübung einer Korrekturkraft zur Erzielung einer therapeutischen Wirkung auf Zehen- und/oder Mittelfußfehlstellungen verwendet werden können, weil beide Schlaufenabschnitte unabhängig voneinander hinsichtlich ihrer Umschlingungslänge einstellbar ausgebildet sind.

Vorzugsweise weist die Hallux-Valgus-Sandale ein Sohlenelement mit Laufsohle und Fußbett, einen Großzehenschlaufenabschnitt zur lateralen Lagefestlegung des Großzehs und einen Mittelfußschlaufenabschnitt auf, welcher beim Tragen der Sandale die Fußmittelknochen eines Fußes, welcher auf dem Sohlenelement getragen ist, zirkulär umschließt, wobei der Mittelfußschlaufenabschnitt in seiner Umschlingungslänge des Fußes veränderbar ist. Durch eine Veränderung der Umschlingungslänge des Mittelfußschlaufenabschnittes bei beispielsweise Verkürzung der Umschlingungslänge des Mittelfußschlaufenabschnittes erhöht sich die Korrekturkraft in Richtung auf die Fußaußenseite, was bereits aus anatomischen Gründen auch eine positive Wirkung in der Ausrichtung der Großzehe aus der Fehlstellung zur Fußaußenseite in Richtung auf eine korrekte Stellung zur Fußinnenseite hin korrigiert. Erfindungsgemäß sind nun Mittelfußschlaufenabschnitt und Großzehenschlaufenabschnitt miteinander gekoppelt, so dass bei Betätigung des Mittelfußschlaufenabschnittes, d. h. bei Veränderung von dessen Umschlingungslänge, auch der Großzehenschlaufenabschnitt im Sinne einer Straffung oder Lockerung betätigt wird, so dass der Großzehenschlaufenabschnitt gleichzeitig mit Straffung oder Lockerung des Mittelfußschlaufenabschnittes eine stärkere oder schwächere Korrekturkraft in Richtung auf die Fußinnenseite ausübt. Die Kopplung zwischen Mittelfußschlaufenabschnitt und Großzehenschlaufenabschnitt erfolgt dabei im Innern der Laufsohle oder zwischen Fußbett und Laufsohle, wenn das eigentliche Fußbett in einer aus der Sandale herausnehmbaren Einlegesohle vorgesehen ist. Der in das Innere der Laufsohle eintauchende Teil des Mittelfußschlaufenabschnittes ist dabei vorzugsweise diagonal mit dem Großzehenschlaufenabschnitt im Innern der Sandale verbunden und tritt aus der Laufsohle oder zwischen Fußbett und Laufsohle wieder aus und bildet dabei eine Schlaufe, welche für die laterale Lagefestlegung des Großzehs vorgesehen und geeignet ist, über welche die Korrekturkraft auf den Großzeh in Richtung auf die Fußinnenseite einstellbar ausübbar ist. Das Material des Mittelfußschlaufenabschnittes wie auch des Großzehenschlaufenabschnittes ist dabei im Wesentlichen zugstarr, so dass die Teile der Schlaufenabschnitte am Mittelfußbereich wie auch am Großzehenbereich jeweils ihre zuvor beschriebene Korrekturkraft ausüben können.

Damit ist es nicht nur möglich, gänzlich auf ein Druckkissen, wie es im Stand der Technik beschrieben und zwingend vorgesehen ist, zu verzichten. Die erfindungsgemäße Sandale kann dadurch aber auch leichter in eine modische Form gebracht werden, ohne dass der therapeutische Effekt der erfindungsgemäßen Sandale negativ beeinflusst wird.

Der Mittelfußschlaufenabschnitt weist ein fußinnenseitiges und ein fußaußenseitiges Teil auf, wobei das fußinnenseitige Teil des Mittelfußschlaufenabschnittes mit dem Großzehenschlaufenabschnitt gekoppelt ist und erfindungsgemäß im Innern der Laufsohle angeordnet ist. Das bedeutet, dass der Mittelfußschlaufenabschnitt in die Laufsohle seitlich eintritt, durch die Mittelfußsohle im Innern hindurchgeführt ist und im Bereich der Großzehe wieder austritt, um dort die Schlaufe des Großzehenschlaufenabschnittes zu dessen lateraler Lagefestlegung zu bilden. Vorzugsweise tritt der Mittelfußschlaufenabschnitt so in die Laufsohle seitlich in einen Kanal ein, dass dieser den Großzehenschlaufenabschnitt im Eintrittsbereich der Laufsohle außen überdeckt oder dass der Großzehenschlaufenabschnitt den Mittelfußschlaufenabschnitt im Eintrittsbereich der Laufsohle außen überdeckt. Die Umschlingungslänge des Mittelfußschlaufenabschnittes wird vorzugsweise durch Ziehen des fußinnenseitigen Teils des Mittelfußschlaufenabschnittes über dessen fußaußenseitigen Teil verkürzt, wodurch die Korrekturkraft beispielsweise vergrößert werden kann bzw. der Mittelfußschlaufenabschnitt und damit auch der Großzehenschlaufenabschnitt entsprechend der therapeutischen Korrektur nachgestellt werden können.

Gemäß einem weiteren Ausführungsbeispiel ist es jedoch auch möglich, dass die Umschlingungslänge des Mittelfußschlaufenabschnittes durch Falten desselben verkürzbar ist. An dem Mittelfußschlaufenabschnitt, welcher im Wesentlichen geschlossen ausgebildet ist, ist eine Einrichtung vorgesehen, mittels welcher eine Auffaltung des Mittelfußschlaufenabschnittes über einen darunterliegenden Bereich und dessen Fixierung in der aufgefalteten Art erfolgt, so dass sich die Länge des Mittelfußschlaufenabschnittes ebenfalls verkürzt.

Vorzugsweise weist das Fußbett im Bereich des Mittelfußschlaufenabschnittes an seiner der Fußinnenseite zugewandten Seite einen hochgezogenen Rand auf, welcher bei Verkürzung der Umschlingungslänge entweder durch Übereinanderziehen eines geteilten Mittelfußschlaufenabschnittes oder durch Auffalten bzw. Zusammenfalten eines geschlossen ausgebildeten Mittelfußschlaufenabschnittes in der Art eines Druckkissens die Korrekturkraft auf den Mittelfußknochen ausübt. Ein Druckkissen selbst ist jedoch nicht erforderlich; durch die spezielle Gestaltung des Fußbettes kann jedoch die Wirkung in der Art eines Druckkissens erzielt werden.

Es ist jedoch gemäß einer Weiterbildung auch möglich, dass der Mittelfußschlaufenabschnitt im Bereich seiner Fußinnenseite eine Pelotte in der Art eines Druckkissens aufweist, welche bei Verkürzung der Umschlingungslänge die Korrekturkraft auf den Mittelfußknochen zusätzlich erhöht. Vorzugsweise ist die Pelotte mittels einer geeigneten lösbaren Verbindung abnehmbar und wieder befestigbar, so dass je nach therapeutischem Heilungserfolg die Größe der Pelotte und damit die zusätzlich ausgeübte Korrekturkraft beeinflussbar sind.

Gemäß einem Ausführungsbeispiel ist an dem Mittelfußschlaufenabschnitt zur Verkürzung seiner Umschlingungslänge ein Drehknopf angeordnet, welcher mit einem Zugelement wie z. B. einem Seil, Kabel oder Draht etc. zusammenwirkt, dessen beide Enden am Mittelfußschlaufenabschnitt im Bereich seiner Befestigung an der Fußaußenseite der Laufsohle befestigt ist. Bei Drehen des Drehknopfes wird dabei das Seil auf einem Aufwickelabschnitt wie einer Walze aufgewickelt, was die wirksame Umschlingungslänge des Mittelfußschlaufenabschnittes verkürzt. Über das Drehen des Druckknopfes kann sowohl eine Verkürzung als auch eine Verlängerung der Umschlingungslänge des Mittelfußschlaufenabschnittes erfolgen, und zwar je nach therapeutischem Behandlungserfolg. Die Verkürzung der Umschlingungslänge kann beispielsweise auch durch den Patienten vorgenommen und eingestellt werden, wenn dieser beispielsweise feststellt, dass bei Beginn der Behandlung mit der erfindungsgemäßen Sandale eine zu große Korrekturkraft ausgeübt wird, welche gegebenenfalls Schmerzen verursachen könnte.

Eine Kopplung zwischen dem Mittelfußschlaufenabschnitt und dem Großzehenschlaufenabschnitt kann nun entweder dergestalt erfolgen, dass der Mittelfußschlaufenabschnitt integral mit dem Großzehenschlaufenabschnitt verbunden und durch das Innere des Sohlenelementes hindurchgeführt ist, so dass bei Veränderung der Überlappung des fußinnenseitigen Teils und des fußaußenseitigen Teils des Mittelfußschlaufenabschnittes gleichzeitig auch ein Straffen des Großzehenschlaufenabschnittes erfolgt, so dass dessen auf den Großzeh ausgeübte Korrekturkraft vergrößert werden kann. Bei Vergrößerung der Umschlingungslänge des Mittelfußschlaufenabschnittes kann die Korrekturkraft, welche auf den Großzeh wirkt, entsprechend verringert werden.

Gemäß einem zweiten Ausführungsbeispiel kann eine Spanneinrichtung, welche als Drehknopf ausgebildet ist, auf dem fußinnenseitigen Teil des Mittelfußschlaufenelementes angeordnet und über ein Zugelement in Form eines Seiles, Kabels oder Drahtes oder dergleichen verbunden sein, welches Zugelement durch das Innere des Sohlenelementes bis hin zu dem der Verankerung des Großzehenschlaufenabschnittes gegenüberliegenden Ende im Inneren des Sohlenelementes geführt ist, so dass eine Verbindung, aber keine integrale Verbindung zwischen den Schlaufenabschnitten des Großzehs und des Mittelfußbereiches vorhanden ist, die Kopplung also über das Zugelement erfolgt. Bei einer Verkürzung des Zugelementes durch Aufwickeln mittels des Drehknopfes würden sich also die Umschlingungslänge des Mittelfußschlaufenabschnittes ebenso wie die Umschlingungslänge des Großzehenschlaufenabschnittes verändern. Die Kopplung zwischen dem Mittelfußschlaufenabschnitt und dem Großzehenschlaufenabschnitt hat somit den wesentlichen Vorteil, dass mit einer einzigen Verstelleinrichtung die zur therapeutischen Wirkung erforderliche Korrekturkraft sowohl im Mittelfußbereich als auch im Großzehenbereich durch eine einzige Verstellung verändert werden kann. Unter Zugelement soll hier ein auf Zug belastbares Element verstanden werden, das auch über eine Eigensteifigkeit verfügt, so dass beim Vergrößern der Umschlingungslänge eine Schiebekraft auf den jeweiligen Schlaufenabschnitt ausübbar ist.

Die Schlaufenabschnitte sind vorzugsweise flexibel und zugstarr, vorzugsweise aus textilem Material oder aus einem mehrschichtigen Kompositmaterial ausgebildet.

Vorzugsweise ist gegebenenfalls entweder nur ein Spannelement für den Großzehenschlaufenabschnitt zur Einstellung von dessen auf den Großzeh wirkenden Korrekturkraft vorhanden oder ist zusätzlich noch ein zweites, gegebenenfalls auch als Drehknopf ausgebildetes Spannelement vorgesehen, welches zur Einstellung der auf den Mittelfußbereich wirkenden Korrekturkraft durch Veränderung des Mittelfußschlaufenabschnittes in seiner Umschlingungslänge realisiert wird. Der wesentliche Vorteil besteht dabei darin, dass die auf den Großzeh oder auf den Mittelfußbereich jeweils ausgeübten Korrekturkräfte einzeln und damit unabhängig voneinander einstellbar sind. Dabei sind zwar, wenn man beide Bereiche hinsichtlich der zur Therapie wirkenden Korrekturkraft einstellen möchte, zwei Spannelemente erforderlich, der wesentliche Vorteil besteht aber in der Unabhängigkeit der Einstellung für die unterschiedlich zu behandelnden Bereiche am Fuß.

Der Mittelfußschlaufenabschnitt ist als geteilter Abschnitt vorgesehen und weist ein fußinnenseitiges Teil und ein fußaußenseitiges Teil auf, welche Teile vorzugsweise einen gewissen Grad an Überlappung bzw. Überdeckung zueinander aufweisen. Durch eine Veränderung der Überlappung bzw. Überdeckung der beiden Enden der beiden Teile des Mittelfußschlaufenabschnittes kann dessen Umschlingungslänge verändert werden. Dies kann vorzugsweise ebenfalls durch ein entsprechend ausgebildetes Spannelement erfolgen, wenn das Zugelement mit dem Drehknopf bzw. der daran angeordneten Aufwickelwalze auf dem einen Abschnitt und die Enden des Zugelementes mit dem anderen Abschnitt des Mittelfußschlaufenabschnittes verbunden sind.

Gemäß einer Weiterbildung der Erfindung kann die Umschlingungslänge des Mittelfußschlaufenabschnittes auch durch Falten oder Entfalten dieses Abschnittes verändert werden.

Wenn gemäß einer Weiterbildung die Kopplung zwischen dem Großzehenschlaufenabschnitt und dem Mittelfußschlaufenabschnitt über eine einteilige Verbindung des beispielsweise fußaußenseitigen Teiles des Mittelfußschlaufenabschnittes und dem Großzehenschlaufenabschnitt realisiert ist, ist für die Verstellung der Umschlingungslänge sowohl des Mittelfußschlaufenabschnittes als auch des Großzehenschlaufenabschnittes nur eine Spanneinrichtung nötig.

Der Drehknopf, welcher wesentlicher Teil der Spanneinrichtung ist, ist vorzugsweise so ausgebildet, dass er einen Wickelabschnitt aufweist, auf welchem das Zugelement zu dessen Verkürzung aufwickelbar ist, wobei zu dessen Verlängerung ein Abwickeln von der Aufwickelwalze erfolgt, und ist außerdem so augebildet, dass dieser in der jeweils eingestellten, eine definierten Umschlingungslänge entsprechenden Aufwickelposition verrastbar ist. Das Verrasten hat den Vorteil, dass beim Tragen der erfindungsgemäßen Hallus-Valgus-Sandale ein unbeabsichtigtes Lösen und damit Verlieren der therapeutischen Wirkung der durch die Verringerung der Umschlingungslänge erhöhten Korrekturkraft verhindert wird. Anstelle des Drehknopfes kann auch eine Klemmeinrichtung vorgesehen sein, mittels welcher das Zugelement in einer gewünschten Position festlegbar bzw. fixierbar ist.

Weitere Vorteile, Ausgestaltungen und Details der Erfindung werden nun anhand eines Ausführungsbeispiels unter Bezugnahme auf die Zeichnung beschrieben. In der Zeichnung zeigen:
- Figur 1: eine perspektivische Darstellung mit Blickrichtung schräg von oben auf die erfindungsgemäße Sandale;
- Figur 2: eine perspektivische Ansicht als Draufsicht auf die Sandale gemäß Figur 1;
- Figur 3: eine perspektivische Darstellung als Seitenansicht auf die Fußaußenseite der erfindungsgemäßen Sandale;
- Figur 4: eine perspektivische Ansicht schräg von oben in Richtung auf die Fußinnenseite der erfindungsgemäßen Sandale;
- Figur 5: eine perspektivische Ansicht schräg von oben in Richtung auf die Fußaußenseite für ein zweites Ausführungsbeispiel gemäß der Erfindung;
- Figur 6: eine prinzipielle Ansicht eines Fußes auf dem Sohlenelement gemäß einem weiteren Ausführungsbeispiel ohne Darstellung der Schlaufenabschnitte;
- Figur 7: eine perspektivische Darstellung als Seitenansicht auf die Fußinnenseite eines weiteren Ausführungsbeispiels der erfindungsgemäßen Sandale;
- Figur 8: eine perspektivische Darstellung als Draufsicht auf das Ausführungsbeispiel gemäß Figur 7; und
- Figur 9: eine perspektivische Draufsicht eines weiteren Ausführungsbeispiels.

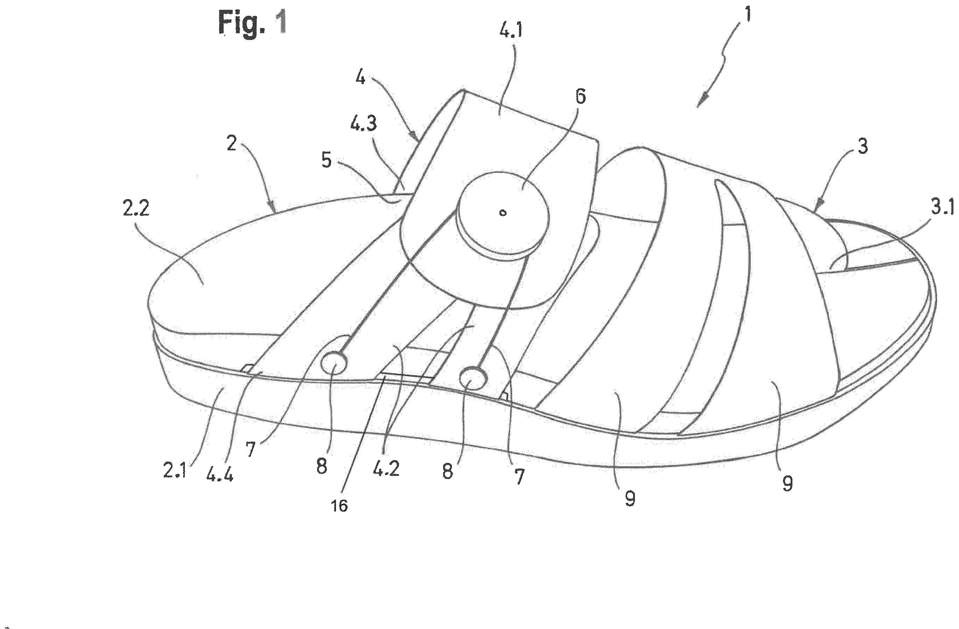

In Figur 1 ist eine perspektivische Ansicht der erfindungsgemäßen Sandale in einer Blickrichtung schräg von oben von der Fußaußenseite dargestellt. Die Hallux-Valgus-Sandale 1 weist in an sich bekannter Bauart ein zweigeteiltes Sohlenelement 2 auf, das aus eigentlicher Laufsohle 2.1 und Fußbett 2.2 besteht, wobei das Fußbett in einer auf der Laufsohle 2.1 im Innern der Sandale 1 angeordneten Einlegesohle ausgebildet ist. Das Fußbett 2.2 kann auch mit der Laufsohle 2.1 fest verbunden, insbesondere verklebt sein.

Im Grundaufbau weist die erfindungsgemäße Sandale 1 einen Großzehenschlaufenabschnitt 3 und einen Mittelfußschlaufenabschnitt 4 auf. Der Mittelfußschlaufenabschnitt 4 ist geteilt und weist ein fußinnenseitiges Teil 4.1 und ein fußaußenseitiges Teil 4.2 auf. Das fußinnenseitige Teil 4.1 ist in übergreifender Art auf dem fußaußenseitigen Teil 4.2 angeordnet, d. h. überlappt letzteres. Zwischen dem Großzehenschlaufenabschnitt 3 und dem Mittelfußschlaufenabschnitt 4 sind zwei Zwischenschlaufenabschnitte 9 vorgesehen, welche den sandalenartigen Charakter dieses dennoch therapeutischen Schuhs unterstreichen sollen, welche aber für den therapeutischen Erfolg des Hallux-Valgus ohne Belang sind. Bei beispielsweise nicht vorhandenem Mittelfußschlaufenabschnitt 4 dienen die Zwischenschlaufabschnitte als Halteschlaufabschnitte. Der Mittelfußschlaufenabschnitt 4 kann in seiner den Mittelfußbereich umgebenden Anordnung in seiner Umschlingungslänge eingestellt bzw. angepasst werden. Die Umschlingungslänge wird dabei durch die beiden sich einander überlappenden Teile 4.1 und 4.2 des Mittelfußschlaufenabschnittes 4 sowie das Fußbett in der Einlegesohle unter einem in der Sandale befindlichen Fuß gebildet. Durch einen Drehknopf 6, welcher über eine Zugelementverbindung in Form eines Seiles mit Befestigungen 8 zusammenwirkt, wobei der Drehknopf an dem fußinnenseitigen Teil 4.1 und die Befestigungen 8 an dem fußaußenseitigen Teil der erfindungsgemäßen Sandale angeordnet sind, verkürzt sich oder verlängert sich bei Betätigung des Drehknopfes 6, und zwar je nach Drehrichtung des Drehknopfes, die Länge des Seiles und damit die Länge der Überlappung des fußinnenseitigen Teils 4.1 über dem fußaußenseitigen Teil 4.2, so dass sich bei einer Verringerung dieser Länge des Zugelements 7 die Umschlingungslänge des Mittelfußschlaufenabschnittes 4 um den Mittelfußbereich verringert.

Das fußaußenseitige Teil 4.2 des Mittelfußschlaufenabschnittes 4 ist im Innern der Sandale 1, d. h. wie in Figur 1 dargestellt, entweder in einem Bereich zwischen der Einlegesohle mit dem Fußbett und der eigentlichen Laufsohle oder, sofern die eigentliche Laufsohle und das Fußbett 2.2 einteilig ausgebildet sind, unterhalb der Einlegesohle mit dem Großzehenschlaufenabschnitt 3 verbunden. D. h., das fußaußenseitige Teil 4.2 des Mittelfußschlaufenabschnittes 4 läuft in einem Kanal 16 im Innern der Sandale 1 von der Außenseite der Sandale, in welche das fußaußenseitige Teil 4.2 des Mittelfußschlaufenabschnittes 4 eintritt, bis zur gegenüberliegenden Seite der Sandale 1, d. h. es tritt an deren Fußinnenseite wieder aus dem Sohlenelement aus. Der andere Endbereich 3.2 des dadurch gebildeten Schlaufenabschnittes für den Großzeh ist also an der Fußinnenseite ausgebildet. Das Ende 4.4 dieses einen Teils des Mittelfußschlaufenabschnittes 4 und des Großzehenschlaufenabschnittes 3, und zwar das am Großzehenschlaufenabschnitt befindliche Ende, ist an der eigentlichen Laufsohle 2.1 der Sandale 1 befestigt. Dort ist dieser erste Endbereich 3.1 des Großzehenschlaufenabschnittes 3 in der Laufsohle 2.2 verankert, während sein zweiter Endbereich 3.2 im Kanal 16 im Innern des Sohlenelementes 2 in den fußaußenseitigen Teil 4.2 des Mittelfußschlaufenabschnittes 4 übergeht. Auch aus Gründen der Montage ist das Ende des Großzehenschlaufenabschnittes 3 an der eigentlichen Laufsohle 2.1 festgelegt, so dass sich zur Fußinnenseite der Sandale 1 die eigentliche Schlaufe des Großzehenschlaufenabschnittes 3 ausbildet.

Das Fußbett 2.2 weist an seinem der Fußinnenseite gerichteten Fußgewölbe einen erhöhten Rand auf, welcher beim Verkürzen der Umschlingungslänge des Mittelfußschlaufenabschnittes 4 zu einer vergrößerten Korrekturkraft auf die Mittelfußknochen führt. Es ist jedoch auch möglich, dass anstelle des erhöhten Randes 5 eine separate Pelotte angeordnet ist, welche die gleiche Funktion einer Korrekturkraft auf die Mittelfußknochen zur unterstützenden Korrektur der Fehllage des Großzehs bei Verkürzung der Umschlingungslänge des Mittelfußschlaufenabschnittes 4 hat.

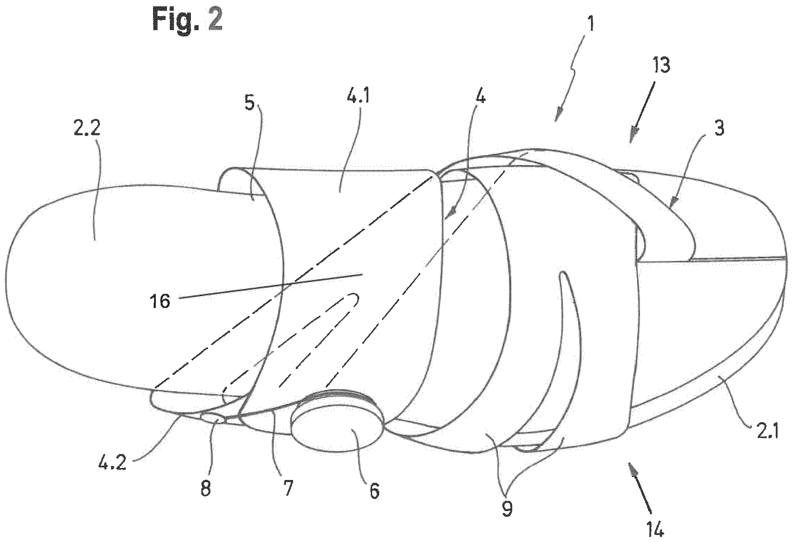

In Figur 2 ist eine perspektivische Draufsicht auf die erfindungsgemäße Sandale gemäß Figur 1 gezeigt, bei welcher schematisch die unter dem Fußbett 2.2 zwischen der Unterseite des Fußbettes und der eigentlichen Laufsohle 2.1 die integrale Kopplung zwischen dem Mittelfußschlaufenabschnitt 4 und dem Großzehenschlaufenabschnitt 3 durch die gestrichelten Linien eingezeichnet ist. Diese gestrichelten Linien deuten auch die Lage und Form des Kanals 16 an, der im Mittelfußbereich von der Fußinnenseite zur Fußaußenseite führt. Das fußaußenseitige Teil 4.2 ist im Kanal 16 unter dem Fußbett 2.2 durch die Sandale hindurchgeführt und tritt auf der fußinnenseitigen Seite wieder aus und bildet die Schlaufe für den Großzehenschlaufenabschnitt 3. Auf dem fußinnenseitigen Teil 4.1 des Mittelfußschlaufenabschnittes 4 ist der Drehknopf 6 angeordnet, auf welchem Zugelemente 7 in Form von Seilen so mit Befestigungen 8 an den fußaußenseitigem Teil 4.2 des Mittelfußschlaufenabschnittes 4 befestigt sind, dass durch Drehen des Drehknopfes 6 die Zugelemente 7 aufgewickelt oder abgewickelt werden, wodurch sich die Umschlingungslänge des Mittelfußschlaufenabschnittes 4 verkürzt oder bei Drehung in die Gegenuhrzeigerrichtung verlängert. Dadurch kann der auf dem Mittelfußknochen wirkende Druck und damit die Korrekturkraft und damit die therapeutische Wirkung eingestellt werden. Das fußinnenseitige Teil 4.1 übergreift den hochgezogenen Rand 5 des Fußbettes 2.2, so dass sich bei Verkürzung der Umschlingungslänge des Mittelfußschlaufenabschnittes 4 die auf den zur Fußinnenseite gerichteten Mittelfußknochen wirkende Korrekturkraft vergrößert. Gleichzeitig wird bei Verkürzung der Umschlingungslänge durch die Kopplung des Mittelfußschlaufenabschnittes 4 mit dem Großzehenschlaufenabschnitt 3 auch der Großzehenschlaufenabschnitt gestrafft, so dass auch auf den Großzeh eine entsprechende Korrekturkraft ausgeübt wird. Der sonstige Grundaufbau entspricht dem der in Figur 1 beschriebenen Ansicht. Der fußaußenseitige Teil 4.2 des Mittelfußschlaufenabschnittes 4, welcher die eigentliche Kopplung zum Großzehenschlaufenabschnitt 3 realisiert, besteht an seinem in Richtung auf den fußinnenseitigen Teil gerichteten Ende aus zwei Enden, welche im Verlauf der Kopplung über eine ausgesparte Zunge wieder zu einem ungeteilten Teilstück zusammenlaufen. Dies ist der Bereich der Kopplung, welcher unmittelbar an den Großzehengurt vor dessen Austritt unter dem Fußbett 2.2 anschließt.

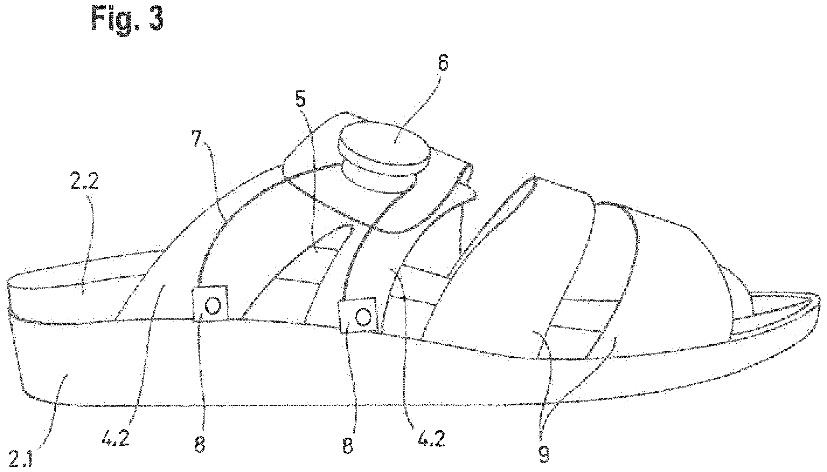

In Figur 3 ist eine perspektivische Seitenansicht der Ausführungsbeispiele gemäß Figur 1 oder Figur 2 dargestellt. Zum einen wird deutlich, dass das Fußbett 2.2 in Form einer Einlegesohle in die Sandale eingelegt ist. Zum anderen ist der fußaußenseitige Teil des Mittelfußschlaufenabschnittes 4 aus zwei Laschen bestehend dargestellt, an welchen jeweils eine Befestigung 8 vorgesehen ist. Von der Befestigung 8 sind Zugelemente 7 an den Drehknopf 6 derart angelenkt, dass beim Drehen des Drehknopfes das Seil sich verkürzen oder verlängern kann, was zu einer Verkürzung der Umschlingungslänge des Mittelfußschlaufenabschnittes 4 führt, oder sich verlängern kann, was zu einer Verlängerung der Umschlingungslänge führt, so dass eine entsprechende therapeutische Wirkung eingestellt werden kann. Bei Verkürzung der Umschlingungslänge erhöht sich die auf den Mittelfußknochen an der Fußinnenseite wirkende Korrekturkraft, während sich diese bei Verlängerung der Umschlingungslänge verringert. Dadurch ist es möglich, dass der Patient je nach gewünschtem Therapieerfolg und nach eingestellter Straffheit der Schlaufenabschnitte über den therapeutischen Erfolg letztendlich auch in gewissem Umfang selbst entscheiden kann. Die Zwischenschlaufenabschnitte 9, welche sich zwischen dem Mittelfußschlaufenabschnitt 4 und dem Großzehenschlaufenabschnitt 3 befinden, verdecken in der Ansicht gemäß Figur 3 den Großzehenschlaufenabschnitt, so dass dieser in der Figur nicht sichtbar ist.

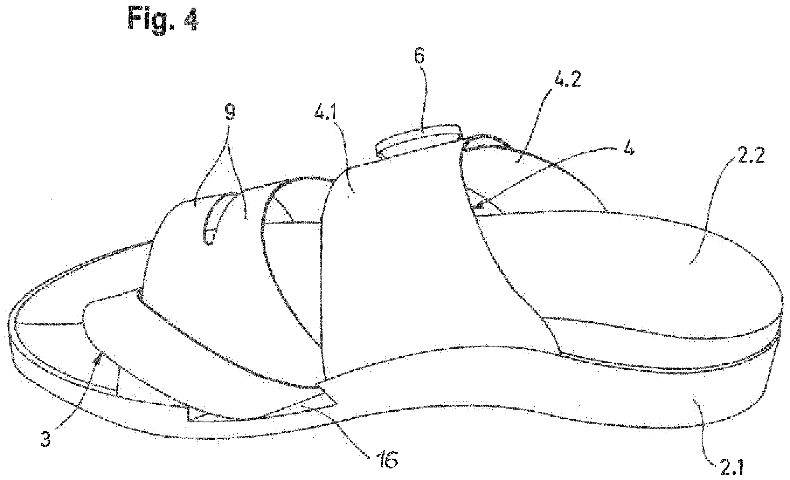

In Figur 4 ist eine perspektivische Seitenansicht von der Fußinnenseite her gezeigt, bei welcher dargestellt ist, wie der Großzehenschlaufenabschnitt 3 in den Kanal 16 im Bereich der Laufsohle 2.1 unterhalb des als Einlegesohle ausgebildeten Fußbettes 2.2 hineingeführt ist. Dieser Großzehenschlaufenabschnitt ist durch die Kopplung direkt mit dem fußaußenseitigen Teil 4.2 des Mittelfußschlaufenabschnittes verbunden, so dass das Drehen des Drehknopfes 6 zu einer Veränderung der Umschlingungslänge um den Mittelfußbereich und einer gleichzeitigen Straffung sowohl des Mittelfußschlaufenabschnittes 4 als auch des Großzehenschlaufenabschnittes 3 führt, wenn die Umschlingungslänge verringert wird. Ebenso kann die Straffung verringert werden, wenn die Umschlingungslänge vergrößert wird. Ansonsten entspricht diese Darstellung gemäß Figur 4 dem Ausführungsbeispiel gemäß Figur 1.

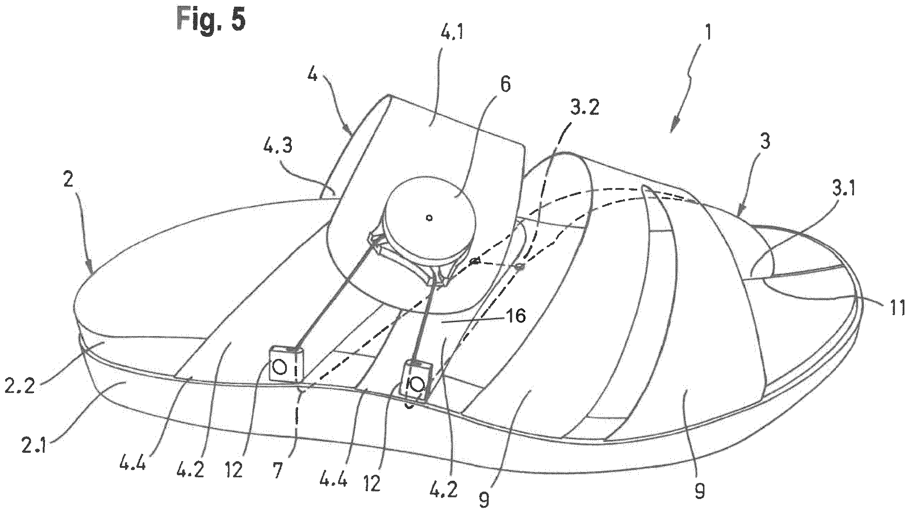

In Figur 5 ist eine perspektivische Darstellung eines zweiten Ausführungsbeispiels gemäß der Erfindung dargestellt mit einer Blickrichtung schräg von oben auf die Außenseite der Sandale, d. h. der Seite der Sandale, an welcher die Fußaußenseite 14 angeordnet ist. Wiederum weist diese Hallux-Valgus-Sandale 1 ein Sohlenelement 2 mit einer eigentlichen Laufsohle 2.1 und einem Fußbett 2.2 auf. Das Fußbett kann mit der eigentlichen Laufsohle 2.1 fest verbunden sein, es ist jedoch auch möglich, dass das Fußbett 2.2 als Einlegesohle in die eigentliche Laufsohle 2.1 eingelegt wird. Aus Gründen der Montage ist es auch denkbar, dass das Fußbett 2.2 in Form der Einlegesohle in die eigentliche Laufsohle 2.1. eingelegt wird und dort dauerhaft verklebt oder anderweitig fest verbunden wird. Die Sandale 1 weist einen geteilten Mittelfußschlaufenabschnitt 4 auf, welcher einen fußinnenseitigen Teil 4.1 und einen fußaußenseitigen Teil 4.2 aufweist. Der fußaußenseitige Teil ist geschlitzt und weist zwei zungenartige Enden auf, welche ebenso wie der ungeschlitzte fußaußenseitige Teil 4.2 des Mittelfußschlaufenabschnittes 4 zwischen dem Fußbett 2.2 und der eigentlichen Laufsohle 2.2 mit der Sandale 1 an ihren Enden befestigt sind. An dem fußinnenseitigen Teil 4.1 des Mittelfußschlaufenabschnittes 4, welches mit seinem Endbereich 4.3 am Sohlenelement 2 befestigt ist, ist eine Spanneinrichtung in Form eines Drehknopfes 6 vorgesehen, auf welcher ein Zugelement wie z. B. ein Kabel, Seil oder Garn beim Drehen des Drehknopes aufwickelbar oder abwickelbar ist. Es sind zwei derartige Zugelemente vorhanden, welche von jeder Seite des Drehknopes 6 her tangential zu einer unterhalb des Drehknopfes 6 befindlichen Aufwickeleinrichtung führen. Das fußaußenseitige Teil 4.2 des Mittelfußschlaufenabschnittes ist mit seinem Endbereich 4.4 am Sohlenelement 2 befestigt.

Im Bereich der Verbindungsstelle zwischen dem Fußbett 2.2 und der eigentlichen Laufsohle 2.1 des Sohlenelementes 2 ist an jeder zungenartigen Lasche des fußaußenseitigen Teils 4.2 des Mittelfußschlaufenabschnittes 4 je eine Umlenkeinrichtung 12 angeordnet, von welcher das jeweils dort umgelenkte Zugelement bzw. das jeweils dort umgelenkte Kabel in den Kanal 16 im Inneren der Sandale 1 geführt ist. In diesem Kanal, dessen Lage in Figur 5 durch die gestrichelten Linien im Sohlenbereich angedeutet ist, ist das jeweilige Ende des Zugelementes 7 mit dem zweiten Endbereich 3.2 des Großzehenschlaufenabschnittes 3 verbunden, welcher von seiner Verankerung 11 um den Großzeh 10 herum bis zu dem Eintritt in das Innere der Sandale 1 an der Fußinnenseite bzw. der Seite der Sandale, welche der Fußinnenseite zugewandt ist, geführt ist, wo die beiden Enden der Zugelemente 7 angelenkt sind. Das bedeutet, dass der zweite Endbereich 3.2 des Großzehenschlaufenabschnittes 3 über die Seile und die Umlenkeinrichtungen 12 zu dem Drehknopf 6 auf dem fußinnenseitigen Teil 4.1 des Mittelfußschlaufenabschnittes 4 geführt sind. Durch Drehen des Drehknopfes 6 wird das jeweilige Zugelement aufgewickelt oder abgewickelt, so dass durch beispielsweise beim Aufwickeln entstehender Zug nicht nur den Grad der Überlappung des fußinnenseitigen Teils 4.1 über den fußaußenseitigen Teil 4.2 des Mittelfußschlaufenabschnittes 4 vergrößert, sondern auch durch diese Vergrößerung der Überlappung und damit der Verringerung der Umschlingungslänge des Mittelfußschlaufenabschnittes 4 erreicht wird, mit der Folge einer Straffung des Großzehenschlaufenabschnittes 3 und damit einer Vergrößerung der Korrekturkraft auf den Großzeh 10.

Bei einem nicht dargestellten Ausführungsbeispiel ist die Kopplung zwischen Mittelfußschlaufenabschnitt 4 und Großzehenschlaufenabschnitt 3 gemäß den Figuren 1 bis 5 bzw. mittels eines zusätzlichen Zugelementes gemäß Figur 5 nicht gegeben. Vielmehr weist sowohl der Mittelfußschlaufenabschnitt 4 als auch der Großzehenschlaufenabschnitt 3 jeweils eine eigene Spannvorrichtung in Form vorzugsweise eines Drehknopfes 6 auf, so dass bei diesem nicht dargestellten Ausführungsbeispiel die Verstellung der Korrekturkraft individuell entweder im Großzehenbereich oder im Mittelfußbereich oder in beiden Bereichen erfolgen kann.

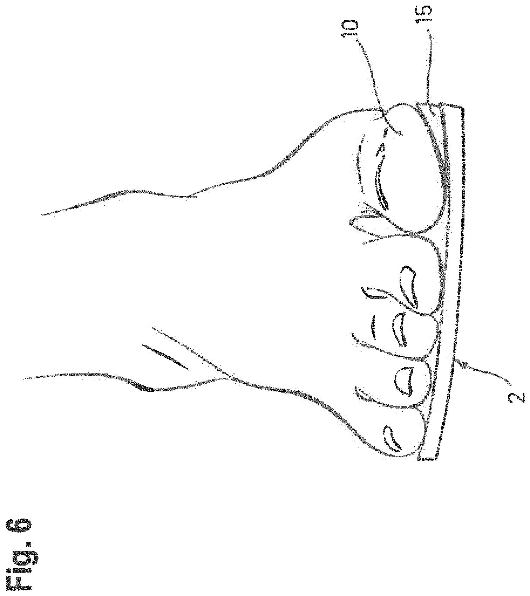

In Figur 6 ist eine Ansicht eines Fußes von vorne auf das Sohlenelement 2 gezeigt, wobei zur Vereinfachung der Darstellung die Schlaufenabschnitte weggelassen sind. Der Großzeh 10 ruht auf einer zur Fußinnenseite sich in Richtung des Zehs erstreckenden keilförmigen Erhöhung, welche den Großzeh zusätzlich abstützt, wenn dieser durch den Großzehenschlaufenabschnitt 3 die Großzehe 10 zirkulär umfängt. Wenn die Umschlingungslänge des Großzehenschlaufenabschnittes 3 verkürzt wird und damit die Korrekturkraft auf den Großzeh 10 sich erhöht, wird durch die keilförmige Erhöhung 15 während der therapeutischen Behandlung durch das Aufbringen der Korrekturkraft durch den Großzehenschlaufenabschnitt die Längsverdrehung des Großzehs 10 verhindert oder dieser zumindest entgegengewirkt. Die keilförmige Erhöhung 15 dient also der Unterlage oder seitlichen Auflage des Großzehs 10 und ist quer zur Fußlängsrichtung geneigt, steigt also in medialer Richtung an und fällt in lateraler Richtung ab. Die keilförmige Erhöhung 15 kann dabei entweder Teil des Fußbettes 2.2 des Sohlenelementes 2 sein oder auf diesem auf dessen Oberseite mit entsprechender Befestigung insbesondere Verklebung aufgebracht sein.

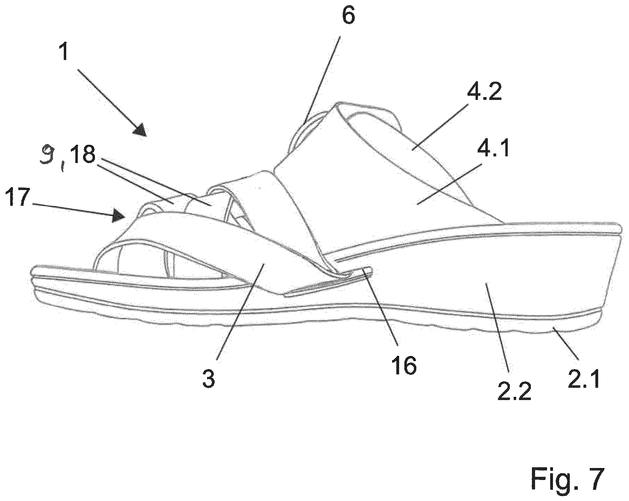

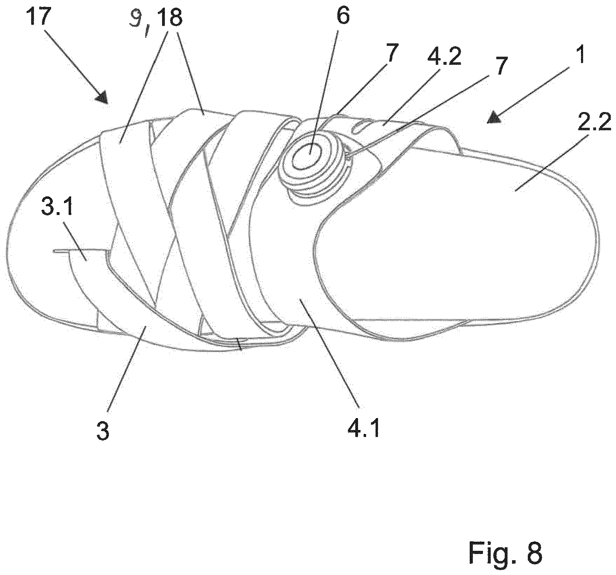

Figur 7 ist eine perspektivische Darstellung als Seitenansicht auf die Fußinnenseite eines weiteren Ausführungsbeispiels der erfindungsgemäßen Sandale 1 gezeigt. Diese Sandale 1 weist eine Laufsohle 2.1 auf, auf der ein Fußbett 2.2 angeordnet ist. Auf dem Fußbett 2.2 ist im Mittelfußbereich eine quer über die Sandale 1 verlaufende aus mehreren Bändern bestehende Bänderung 17 vorhanden, von denen einige Bänder 18 fest mit dem Fußbett 2.2 verbunden sind. Der Großzehenschlaufenabschnitt 3 ist mit seinem ersten Endbereich 3.1 am Fußbett 2.2 verankert und schräg nach hinten zum Kanal 16 geführt. Dort tritt er über den Kanal 16 in das Innere des Fußbettes 2.2 ein, wo er sich mit dem Mittelfußschlaufenabschnitt 4 vereinigt, um gemeinsam mit diesem auf der anderen Seite des Fußbetts 2.2 auszutreten, oder an ein je ein Zugelement 7 angekoppelt zu werden, wie in Figur 8 gezeigt ist. Die Spannung des Großzehenschlaufenabschnitts 3 und des Mittelfußschlaufenabschnitts 4 erfolgt wie zuvor über Zugelemente 7, die mittels des Drehknopfs 6 gestrafft oder gelockert werden können.

Figur 8 zeigt die in Figur 7 dargestellte Sandale 1 als perspektivische Darstellung von oben.

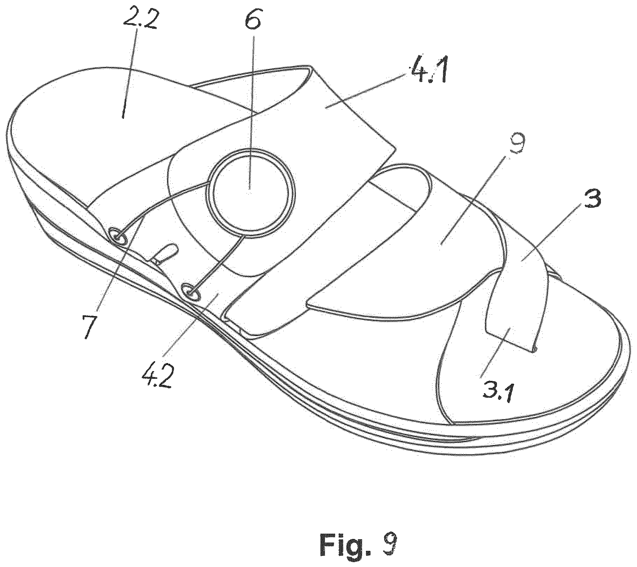

Figur 9 zeigt ein weiteres Ausführungsbeispiel einer erfindungsgemäßen Sandale, deren Grundaufbau dem gemäß Figur 7 bzw. Figur 8 entspricht, bei welcher jedoch der Mittelfußschlaufenabschnitt 4 nicht als Bänderung ausgebildet ist, sondern zwei breitere Laschen aufweist, welche übereinanderlegbar und mit einem Klettverschluss in der gewünschten Schlaufengröße anpassbar sind. Die Sandale weist ebenso ein Fußbett 2.2 auf, auf welchem im Mittelfußbereich neben dem Mittelfußschlaufenabschnitt 4 in der beschriebenen Ausgestaltung außerdem ein Zwischenschlaufenabschnitt 9 angeordnet ist, wobei der Mittelfußschlaufenabschnitt 4 ein fußinnenseitiges Teil 4.1 und ein fußaußenseitiges Teil 4.2 aufweist, welche mittels eines Drehknopfes 6 und einem Zugelement 7 zueinander verstellbar angeordnet sind, wobei ein Großzehenschlaufenabschnitt 3 mit einem ersten Endbereich 3.1 am Fußbett 2.2 verankert und schräg nach hinten zu dem in Figur 9 nicht dargestellten Kanal 16 geführt ist. Dort tritt er über den Kanal in das Innere des Fußbettes 2.2 ein, wo er mit dem Mittelfußschlaufenabschnitt 4 bzw. 4.2 vereinigt ist, um gemeinsam mit diesem auf der anderen Seite des Fußbettes 2.2 auszutreten. Es ist jedoch auch möglich, dass der Großzehenschlaufenabschnitt 3 an je ein Zugelement angekoppelt ist, wie es in Figur 8 gezeigt ist. Die Spannung des Großzehenschlaufenabschnittes 3 und des Mittelfußschlaufenabschnittes 4 erfolgt wie zuvor über Zugelemente 7, die mittels des Drehknopfes 6 gestrafft oder gelockert werden können.

Im Bereich zwischen dem Mittelfußabschnitt 4 und dem Großzehenschlaufenabschnitt 3 ist ein Zwischenschlaufenabschnitt 9 vorgesehen, welcher als Laschenband mit einem Klettverschluss ausgebildet ist. Das Laschenband wirkt wie die Bänderung 17 gemäß Figuren 7 und 8, jedoch mit sozusagen zu dem Laschenband zusammengefassten Bändern.

### Bezugszeichenliste

- 1: Sandale
- 2: Sohlenelement
- 2.1: Laufsohle
- 2.2: Fußbett
- 3: Großzehenschlaufenabschnitt
- 3.1: erster Endbereich
- 3.2: zweiter Endbereich
- 4: Mittelfußschlaufenabschnitt
- 4.1: fußinnenseitiges Teil
- 4.2: fußaußenseitiges Teil
- 4.3: Endbereich fußinnenseitiger Teil
- 4.4: Endbereich fußaußenseitiger Teil
- 5: hochgezogener Rand
- 6: Drehknopf
- 7: Zugelement
- 8: Befestigung
- 9: Zwischenschlaufenabschnitt
- 10: Großzeh
- 11: Verankerung
- 12: Umlenkeinrichtung
- 13: Fußinnenseite
- 14: Fußaußenseite
- 15: keilförmige Erhöhung
- 16: Kanal
- 17: Bänderung
- 18: feste Bänder

## Patentansprüche

1. Hallux-Valgus-Sandale (1) zum Einsatz bei Fehlstellungen zumindest von Großzehen und Mittelfußknochen, welche aufweist:
ein Sohlenelement (2) mit einer Laufsohle (2.1) und einem beim Tragen in einem Fuß dem Fuß zugeordneten Fußbett (2.2),
einen Mittelfußschlaufenabschnitt (4) zum Halten der Sandale (1) am Fuß und einen Großzehenschlaufenabschnitt (3),
wobei der Großzehenschlaufenabschnitt (3) mit einem ersten Endbereich (3.1) in einem zwischen dem Großzeh und dem benachbarten Zeh gelegenen Bereich des Sohlenelements (2) am oder im Sohlenelement (2) verankert und in seiner Umschlingungslänge um den Großzeh (10) derart veränderbar ist, dass eine einstellbare Korrekturkraft auf den Großzeh (10) ausübbar ist, und
wobei der Großzehenschlaufenabschnitt (3) vom ersten Endbereich (3.1) ausgehend über den Bereich des Großzehs schräg nach hinten geführt ist und auf der Innenseite der Hallux-Valgus-Sandale (1) in einen zur Außenseite der Hallux-Valgus-Sandale (1) führenden Kanal (16) im Sohlenelement (2) eintritt,
**dadurch gekennzeichnet, dass**
- der Mittelfußschlaufenabschnitt (4) am Sohlenelement (2) fixiert ist,
- der Mittelfußschlaufenabschnitt (4) ein fußinnseitiges (4.1) und ein fußaußenseitiges Teil (4.2) aufweist und das fußinnenseitige Teil (4.1) des Mittelfußschlaufenabschnittes (4) mit dem Großzehenschlaufenabschnitt (3) gekoppelt ist, und
- der Kanal (16) im Mittelfußbereich der Hallux-Valgus-Sandale (1) derart hinter dem Großzehenballenbereich eines Fußes angeordnet ist, dass der Großzehenschlaufenabschnitt (3) den fußinnenseitigen Mittelfußschlaufenabschnitt (4.1) im Eintrittsbereich in die Laufsohle (2.1) außen überdeckt.

2. Hallux-Valgus-Sandale nach Anspruch 1, **dadurch gekennzeichnet, dass** der Mittelfuß schlaufenabschnitt (4) an seinen jeweiligen Endbereichen (4.3, 4.4) am oder im Sohlenelement (2) befestigt ist und dessen Umschlingungslänge um den Mittelfußbereich derart veränderbar ist, dass eine einstellbare Korrekturkraft auf den Mittelfußbereich ausübbar ist.

3. Hallux-Valgus-Sandale gemäß Anspruch 2 zum Einsatz bei Fehlstellungen zumindest von Großzeh- und Mittelfußknochen mit einem Sohlenelement (2) mit Laufsohle (2.1) und Fußbett (2.2), einem Großzehenschlaufenabschnitt (3) zur lateralen Lagefestlegung des Großzehs und einem Mittelfußschlaufenabschnitt (4), welcher beim Tragen der Sandale die Fußmittelknochen eines auf dem Sohlenelement (2) getragenen Fußes zirkulär umschließt und in seiner Umschlingungslänge veränderbar ist, wobei
der Mittelfußschlaufenabschnitt (4) und der Großzehenschlaufenabschnitt (3) derart miteinander gekoppelt sind, dass bei Veränderung der Umschlingungslänge des Mittelfußschlaufenabschnittes (4) eine auf die Mittelfußknochen lateral wirkende Mittelfuß-Korrekturkraft variierbar ist, die einen an der Fußinnenseite (13) liegenden Mittelfußknochen in seitlicher Richtung zur Fußaußenseite (14) drückt und durch die bei einer in oder an der Laufsohle angeordneten Kopplung des Mittelfußschlaufenabschnittes (4) mit dem Großzehenschlaufenabschnitt (3) letzterer gleichzeitig mitgestrafft wird, wodurch eine Großzehen-Korrekturkraft in Richtung auf die Fußinnenseite (13) ausübbar ist.

4. Hallux-Valgus-Sandale nach einem der Ansprüche 2 oder 3, **dadurch gekennzeichnet, dass** die Umschlingungslänge durch Ziehen des fußinnenseitigen Teils (4.1) des Mittelfußschlaufenabschnittes (4) über dessen fußaußenseitiges Teil (4, 2) verkürzbar ist.

5. Hallux-Valgus-Sandale nach einem der Ansprüche 2 bis 4, **dadurch gekennzeichnet, dass** die Umschlingungslänge durch Falten des Mittelfußschlaufenabschnittes (4) verkürzbar und durch Entfalten verlängerbar ist.

6. Hallux-Valgus-Sandale nach einem der Ansprüche 2 bis 5, **dadurch gekennzeichnet, dass** das Fußbett (2.2) im Bereich des Mittelfußschlaufenabschnittes (4) an seiner der Fußinnenseite zugewandten Seite einen hochgezogenen Rand (5) aufweist, welcher bei Verkürzung der Umschlingungslänge des Mittelfußschlaufenabschnittes (4) in der Art eines Druckkissens die Korrekturkraft auf den Mittelfußknochen ausübt.

7. Hallux-Valgus-Sandale nach einem der Ansprüche 2 bis 6, **dadurch gekennzeichnet, dass** der Mittelfußschlaufenabschnitt (4) im Bereich seiner Fußinnenseite eine Pelotte in der Art eines Druckkissens aufweist, welches bei Verkürzung der Umschlingungslänge des Mittelfußschlaufenabschnittes (4) die Korrekturkraft auf den Mittelfußknochen ausübt.

8. Hallux-Valgus-Sandale nach einem der Ansprüche 4 bis 7, **dadurch gekennzeichnet, dass** die Veränderung der Umschlingungslänge mittels einer als Drehknopf (6) ausgebildeten Spanneinrichtung und, mit diesem Drehknopf (6) zusammenwirkend, eines Zugelementes (7), insbesondere eines Seiles oder Kabels, erfolgt, dessen eines Ende am Drehknopf (6) am fußinnenseitigen Teil (4.1) des Mittelfußschlaufenabschnittes (4) und dessen anderes Ende mit einem zweiten Endbereich (3.2) des Großzehenschlaufenabschnittes (3) am oder im Sohlenelement (2) verbunden sind, wobei der Großzehenschlaufenabschnitt (3) an seinem dazu gegenüberliegenden ersten Endbereich (3.1) am Sohlenelement (2) verankert ist.

9. Hallux-Valgus-Sandale nach Anspruch 1 oder 2, **dadurch gekennzeichnet, dass** mittels einer als Drehknopf ausgebildeten Spanneinrichtung, welche mit einem Zugelement (7), insbesondere einem Kabel oder einem Seil, verbunden ist, bei dessen Verstellen das Zugelement (7) verlängerbar oder verkürzbar und damit die Umschlingungslänge des jeweiligen Schlaufenabschnittes (3, 4) veränderbar ist.

10. Hallux-Valgus-Sandale nach Anspruch 1, **dadurch gekennzeichnet, dass** der Halteschlaufenabschnitt (4) der Mittelfußschlaufenabschnitt (4) ist, welcher ein fußinnenseitiges Teil (4.1) und ein fußaußenseitiges Teil (4.2) aufweist.

11. Hallux-Valgus-Sandale nach Anspruch 2 oder 10, **dadurch gekennzeichnet, dass** die Umschlingungslänge durch Falten oder Entfalten des Mittelfußschlaufenabschnittes (4) veränderbar ist.

12. Hallux-Valgus-Sandale nach Anspruch 2 oder 3, **dadurch gekennzeichnet, dass** die Kopplung zwischen dem Großzehenschlaufenabschnitt (3) und dem Mittelfußschlaufenabschnitt (4) dadurch erfolgt, dass der Mittelfußschlaufenabschnitt (4) mit dem Großzehenschlaufenabschnitt (3) im Sohlenelement einteilig verbunden ist.

13. Hallux-Valgus-Sandale nach Anspruch 8 oder 9, **dadurch gekennzeichnet, dass** der Drehknopf einen Wickelabschnitt aufweist, auf welchem das Zugelement (7) zu dessen Verkürzung aufwickelbar oder zu dessen Verlängerung von dieser Aufwickelwalze abwickelbar ist und dass der Drehknopf in der jeweils eingestellten, einer definierten Umschlingungslänge entsprechenden Aufwickelposition verrastbar ist.

14. Hallux-Valgus-Sandale nach einem der Ansprüche 1 bis 13, **dadurch gekennzeichnet, dass** im Bereich zwischen dem Mittelfußschlaufenabschnitt (4) und dem Großzehenschlaufenabschnitt (3) ein Zwischenschlaufenabschnitt (9) in Form einer Bänderung (17) oder eines Laschenbandes mit Klettverschluss ausgebildet ist.

## Claims

1. Hallux valgus sandal (1) for use in case of malpositions of at least great toes and metatarsal bones, comprising:
a sole element (2) having an outsole (2.1) and a footbed (2.2) attached to a foot when being worn at the foot,
a metatarsus strap section (4) configured for holding the sandal (1) at the foot, and
a great toe strap section (3), wherein
the great toe strap section (3) has a first end region (3.1) which is fixed to or embedded in the sole element (2) in a region of the sole element (2) arranged between the great toe and the adjacent toe and is variably adjustable in its wrap length around the great toe (10) such that an adjustable corrective force is exertable on the great toe (10), and wherein
the great toe strap section (3) is guided obliquely backwards from the first end region (3.1) over the region of the great toe and, at an inner side of the hallux valgus sandal (1), enters a channel (16) in the sole element which leads to an outside of the hallux valgus sandal (1),
**characterized in that**
- the metatarsus strap section (4) is fixed to the sole element (2),
- the metatarsus strap section (4) comprises a foot inner side part (4.1) and a foot outer side part (4.2), wherein the foot inner side part (4.1) of the metatarsus strap section (4) is coupled to the great toe strap section (3), and
- the channel (16) is arranged behind a metatarsophalangeal joint of the foot in the metatarsus region of the hallux valgus sandal (1) such that the great toe strap section (3) covers the foot inner side metatarsus strap section (4.1) at an entry region into the outsole (2.1).

2. Hallux valgus sandal (1) according to claim 1, **characterized in that** the metatarsus strap section (4), at its respective end regions (4.3, 4.4), is fixed to or in the sole element (2) and the wrap length around the metatarsus region of which is variably adjustable such that an adjustable corrective force is exertable on the metatarsus region.

3. Hallux valgus sandal according to claim 2 for use in case of malpositions of at least the great toes and metatarsal bones, having a sole element (2) with an outsole (2.1) and a footbed (2.2), a great toe strap section (3) for laterally fixing the position of the great toe, and a metatarsus strap section (4) which, in a state in which the sandal is worn, circularly encloses the metatarsal bones of a foot hold on the sole element (2) and which is adjustable in its wrap length, wherein
the metatarsus strap section (4) and the great toe strap section (3) are coupled to one another such that, upon adjusting the wrap length of the metatarsus strap section (4), a metatarsal corrective force acting laterally upon the metatarsal bones is adjustable which presses a foot inner side (13) metatarsal bone in a lateral direction towards the outer side (14) of the foot and which, when a coupling of the metatarsus strap section (4) and the great toe strap section (3) arranged in or on the outsole, the great toe strap section (3) is simultaneously tightened, based on which a great toe correction force is exertable in direction of the inner side (13) of the foot.

4. Hallux valgus sandal according to any one of claims 2 or 3, **characterized in that** the wrap length is decreasable upon pulling the foot inner side part (4.1) of the metatarsus strap section (4) among its foot outer side part (4.2).

5. Hallux valgus sandal according to any one of claims 2 to 4, **characterized in that** the wrap length is decreasable upon folding the metatarsus strap section (4) and increasable upon unfolding the metatarsus strap section (4).

6. Hallux valgus sandal according to any one of claims 2 to 5, **characterized in that** the footbed (2.2), at its side facing the inner side of the foot, has a raised edge (5) in the region of the metatarsus strap section (4) which, upon decreasing the wrap length of the metatarsus strap section (4), exerts the corrective force on the metatarsal bone in the manner of a pressure pad.

7. Hallux valgus sandal according to any one of claims 2 to 6, **characterized in that** the metatarsus strap section (4) has a pad in the region of its foot inner side provided in the form of a pressure pad which exerts the corrective force on the metatarsal bone upon decreasing the wrap length of the metatarsus strap section (4).

8. Hallux valgus sandal according to any one of claims 4 to 7, **characterized in that** the variation of the wrap length is effected by means of a tensioning unit provided in the form of a rotary knob (6) and a tension element (7), in particular a cable or rope, interacting with the rotary knob (6), wherein one end of the tension element (7) is attached to the rotary knob (6) on the foot inner side part (4.1) of the metatarsus strap section (4) and its other end is fixed to a second end region (3.2) of the great toe strap section (3) on or in the sole element (2), and wherein the great toe strap section (3) is fixed to the sole element (2) at its opposing first end region (3.1).

9. A hallux valgus sandal according to claim 1 or 2, **characterized in that** by means of a tensioning unit provided in the form of a rotary knob which is connected to a tension element (7), in particular a cable or a cord, wherein the length of the tension element (7) is increasable or decreasable and thus the wrap length of the respective strap section (3, 4) is adjustable upon actuation of the tensioning unit.

10. Hallux valgus sandal according to claim 1, **characterized in that** the holding strap section (4) is the metatarsus strap section (4) comprising a foot inner side part (4.1) and a foot outer side part (4.2).

11. Hallux valgus sandal according to claim 2 or 10, **characterized in that** the wrap length is adjustable upon folding or unfolding the metatarsus strap section (4).

12. Hallux valgus sandal according to claim 2 or 3, **characterized in that** the coupling of the great toe strap section (3) and the metatarsus strap section (4) is provided by integrally connecting the metatarsus strap section (4) to the great toe strap section (3) in the sole element.

13. Hallux valgus sandal according to claim 8 or 9, **characterized in that** the rotary knob has a winding section on which the tension element (7) is windable to be shorten or lengthen, and that the rotary knob is lockable in a respectively set winding position associated to a predefined wrap length.

14. Hallux valgus sandal according to any one of claims 1 to 13, **characterized in that**, in the region between the metatarsus strap section (4) and the great toe strap section (3), an intermediate strap section (9) is formed in the form of a banding (17) or a flap strap having a hook-and-loop fastener.

## Revendications

1. Sandale pour hallux valgus (1) à utiliser en cas de désaxations au moins de gros orteils et d'os métatarsiens, laquelle présente :
un élément de semelle (2) avec une semelle extérieure (2.1) et une semelle intérieure (2.2) affectée au pied lors du port sur un pied,
une section de boucle métatarsienne (4) pour maintenir la sandale (1) sur le pied, et une section de boucle de gros orteil (3),
dans laquelle la section de boucle de gros orteil (3) est ancrée avec une première zone d'extrémité (3.1) dans une zone de l'élément de semelle (2) située entre le gros orteil et l'orteil adjacent sur ou dans l'élément de semelle (2) et est modifiable dans sa longueur de boucle autour du gros orteil (10) de sorte qu'une force de correction réglable peut être exercée sur le gros orteil (10), et
dans laquelle la section de boucle de gros orteil (3), partant de la première section de boucle de gros orteil (3.1), est guidée en biais vers l'arrière sur la zone du gros orteil et, sur le côté intérieur de la sandale pour hallux valgus (1), entre dans un canal (16) dans l'élément de semelle (2) menant au côté extérieur de la sandale pour hallux valgus (1),
**caractérisée en ce**
- **que** la section de boucle métatarsienne (4) est fixée sur l'élément de semelle (2),
- **que** la section de boucle métatarsienne (4) présente une partie intérieure de pied (4.1) et une partie extérieure de pied (4.2) et la partie intérieure de pied (4.1) de la section de boucle métatarsienne (4) est couplée à la section de boucle de gros orteil (3), et
- **que** le canal (16) dans la zone métatarsienne de la sandale pour hallux valgus (1) est disposé derrière la zone de coussinet de gros orteil d'un pied de sorte que la section de boucle de gros orteil (3) recouvre la section de boucle métatarsienne intérieure de pied (4.1) à l'extérieur dans la zone d'entrée dans la semelle extérieure (2.1).

2. Sandale pour hallux valgus selon la revendication 1, **caractérisée en ce que** la section de boucle métatarsienne (4) est fixée à ses zones d'extrémité respectives (4.3, 4.4) sur ou dans l'élément de semelle (2) et sa longueur de boucle autour de la zone métatarsienne est modifiable de sorte qu'une force de correction réglable peut être exercée sur la zone métatarsienne.

3. Sandale pour hallux valgus selon la revendication 2 à utiliser en cas de désaxations au moins d'os du gros orteil et métatarsiens, avec un élément de semelle (2) avec une semelle extérieure (2.1) et une semelle intérieure (2.2), une section de boucle de gros orteil (3) pour fixer latéralement la position du gros orteil et une section de boucle métatarsienne (4), laquelle, lors du port de la sandale, entoure de manière circulaire les os métatarsiens d'un pied porté sur l'élément de semelle (2) et dont la longueur de boucle est modifiable, dans laquelle
la section de boucle métatarsienne (4) et la section de boucle de gros orteil (3) sont couplées l'une à l'autre de sorte que, lorsque la longueur de boucle de la section de boucle métatarsienne (4) est modifiée, une force de correction métatarsienne agissant latéralement sur les os métatarsiens est variable, qui presse un os métatarsien situé sur le côté intérieur de pied (13) dans une direction latérale vers le côté extérieur de pied (14) et au moyen duquel, dans le cas d'un couplage de la section de boucle métatarsienne (4) avec la section de boucle de gros orteil (3) disposée dans ou sur la semelle extérieure, cette dernière est simultanément serrée, moyennant quoi une force de correction de gros orteil peut être exercée dans la direction du côté intérieur de pied (13).

4. Sandale pour hallux valgus selon l'une quelconque des revendications 2 ou 3, **caractérisée en ce que** la longueur de boucle peut être raccourcie en tirant la partie intérieure de pied (4.1) de la section de la boucle métatarsienne (4) du côté intérieur de pied sur sa partie extérieure de pied (4, 2).

5. Sandale pour hallux valgus selon l'une quelconque des revendications 2 à 4, **caractérisée en ce que** la longueur de boucle peut être raccourcie en pliant la section de boucle métatarsienne (4) et allongée en la dépliant.

6. Sandale pour hallux valgus selon l'une quelconque des revendications 2 à 5, **caractérisée en ce que** la semelle intérieure (2.2) présente, dans la zone de la section de boucle métatarsienne (4), sur son côté tourné vers le côté intérieur de pied, un bord surélevé (5), lequel exerce la force de correction sur l'os métatarsien à la manière d'un coussin de pression lorsque la longueur de boucle de la section de boucle métatarsienne (4) est raccourcie.

7. Sandale pour hallux valgus selon l'une quelconque des revendications 2 à 6, **caractérisée en ce que** la section de boucle métatarsienne (4) présente, dans la zone de son côté intérieur de pied, une pelote à la manière d'un coussin de pression, lequel exerce la force de correction sur l'os métatarsien lorsque la longueur de boucle de la section de boucle métatarsienne (4) est raccourcie.

8. Sandale pour hallux valgus selon l'une quelconque des revendications 4 à 7, **caractérisée en ce que** la modification de la longueur de boucle est effectuée au moyen d'un dispositif de tension sous la forme d'un bouton rotatif (6) et, coopérant avec ce bouton rotatif (6), d'un élément de traction (7), en particulier une corde ou un câble, dont une extrémité est connectée au bouton rotatif (6) sur la partie intérieure de pied (4.1) de la section de boucle métatarsienne (4) et dont l'autre extrémité est connectée à une seconde zone d'extrémité (3.2) de la section de boucle de gros orteil (3) sur ou dans l'élément de semelle (2), dans laquelle la section de boucle de gros orteil (3) est ancrée dans sa première zone d'extrémité opposée (3.1) sur l'élément de semelle (2).

9. Sandale pour hallux valgus selon la revendication 1 ou 2, **caractérisée en ce qu'**au moyen d'un dispositif de tension sous la forme d'un bouton rotatif, qui est connecté à un élément de traction (7), en particulier un câble ou une corde, l'élément de traction (7) peut être allongé ou raccourci lors de son réglage et la longueur de boucle de la section de boucle respective (3, 4) est ainsi modifiable.

10. Sandale pour hallux valgus selon la revendication 1, **caractérisée en ce que** la section de boucle de maintien (4) est la section de boucle métatarsienne (4), laquelle présente une partie intérieure de pied (4.1) et une partie extérieure de pied (4.2).

11. Sandale pour hallux valgus selon la revendication 2 ou 10, **caractérisée en ce que** la longueur de boucle est modifiable en pliant ou dépliant la section de boucle métatarsienne (4).

12. Sandale d'hallux valgus selon la revendication 2 ou 3, **caractérisée en ce que** le couplage entre la section de boucle de gros orteil (3) et la section de boucle métatarsienne (4) est effectué par la section de boucle métatarsienne (4), qui est connectée d'un seul tenant à la section de boucle de gros orteil (3) dans l'élément de semelle.

13. Sandale pour hallux valgus selon la revendication 8 ou 9, **caractérisée en ce que** le bouton rotatif présente une section d'enroulement sur laquelle l'élément de traction (7) peut être enroulé pour le raccourcir ou peut être déroulé de ce rouleau d'enroulement pour le rallonger, et **en ce que** le bouton rotatif peut être bloqué dans la position d'enroulement respectivement réglée correspondant à une longueur de boucle définie.

14. Sandale pour hallux valgus selon l'une quelconque des revendications 1 à 13, **caractérisée en ce qu'**une section de boucle métatarsienne (9) sous la forme d'une sangle (17) ou d'une sangle à rabat avec fermeture autoagrippante est réalisée dans la zone entre la section de boucle métatarsienne (4) et la section de boucle de gros orteil (3).
